# NEW EUROPEAN PATENT SPECIFICATION

(11) **EP 1 951 064 B2**
(45) Date of publication and mention of the opposition decision: **13.05.2020**
(45) Mention of the grant of the patent: 19.09.2012
(21) Application number: 06819464.6
(22) Date of filing: 14.11.2006
(51) Int. Cl.: A23J 3/08, A23L 1/29, A23K 1/16, A23K 1/18, A23L 1/305

(54) **ORAL TOLERANCE PROMOTION WITH GLYCATED PROTEINS**
VERBESSERUNG DER ORALEN TOLERANZ MIT GLYKIERTEN PROTEINEN
PROMOTION DE TOLERANCE ORALE A BASE DE PROTEINES GLYCATEES

(30) Priority: 14.11.2005 EP 05257007
(43) Date of publication of application: 06.08.2008
(73) Proprietor: Société des Produits Nestlé S.A., 1800 Vevey (CH)
(72) Inventor: PECQUET, Sophie, CH-1012 Lausanne (CH); BOVETTO, Lionel Jean René, F-74500 Larringes (FR); MORGAN, François, F-35000 Rennes (FR)
(74) Representative: Elkington and Fife LLP
(86) International application number: PCT/EP2006/068445
(87) International publication number: WO 2007/054587

(56) References cited:
- EP-A- 1 514 482
- WO-A-00/18249
- US-A- 5 750 176
- US-A1- 2003 114 367
- BAGCHI D ET AL: "Effects of orally administered undenatured type II collagen against arthritic inflammatory diseases: A mechanistic exploration." INTERNATIONAL JOURNAL OF CLINICAL PHARMACOLOGY RESEARCH, vol. 22, no. 3-4, 2002, pages 101-110, XP008062718 ISSN: 0251-1649
- KATO CHIAKI ET AL: "The influence of initial exposure timing to beta-lactoglobulin on oral tolerance induction" JOURNAL OF ALLERGY AND CLINICAL IMMUNOLOGY, vol. 104, no. 4 PART 1, October 1999 (1999-10), pages 870-878, XP002376187 ISSN: 0091-6749
- MIZUMACHI KOKO ET AL: "Induction of oral tolerance in mice by continuous feeding with beta-lactoglobulin and milk" BIOSCIENCE BIOTECHNOLOGY AND BIOCHEMISTRY, vol. 66, no. 6, June 2002 (2002-06), pages 1287-1294, XP002376188 ISSN: 0916-8451

## Description

### Field of the invention

The present invention relates to a method of promoting oral tolerance in an animal comprising administering glycated proteins to the animal; glycated proteins for use in the promotion of oral tolerance in an animal; a food product comprising glycated proteins for use in the same, and a method for making said food product.

### Background of the invention

Mother's milk is recommended for all infants. However, in some cases breast feeding is inadequate or unsuccessful or inadvisable for medical reasons or the mother chooses not to breast feed. Infant formulae have been developed for these situations. The majority of infant formulae are based on cows' milk. However, cows' milk allergy or milk hypersensitivity is common in infants. Usually it disappears by the age of two or three years, but it may occasionally be lifelong. It is the most common disease in infants, with an incidence of 0.5 to 3 % in full term infants and 3 to 5 % in pre-term infants. This allergy can cause rashes, hives, redness around the mouth, a runny nose, sneezing, colic, diarrhoea, vomiting, anaphylaxis, or more generally digestive troubles. It could also be associated with some cases of infant sudden death.

Milk hypersensitivity should be distinguished from lactose intolerance, which is intolerance to milk as a result of congenital deficiency of the lactase enzyme.

Cows' milk allergy is caused, in most cases, by a reaction to the α-lactalbumin and β-lactoglobulin proteins in the whey fraction. It can also be caused by casein and/or albumin, which are potentially allergenic lactic proteins also present in cows' milk. In the early months of life the immune system is still developing and may fail to recognise and tolerate such dietary proteins. The result is that the baby treats the dietary protein as a foreign substance and develops an allergy to it. Children may also develop allergies to other dietary proteins if their immune system does not recognise and tolerate these proteins.

There are two types of immune response: cell mediated (or Th1) which is important for attack by bacteria and viruses, and humoral (or Th2) which is implicated in allergic reactions. Before the infant is born, its body is sterile and the Th1 pathway is inactive. The Th2 pathway, however, is active before birth because it is necessary to prevent the baby from setting up an immune response to its mother. Immediately the infant is born, it is exposed to numerous bacteria, both benign and harmful, and the Th1 pathway is activated to deal with this onslaught. As each new potential foreign substance is encountered in the gut, it is taken up by the gut-associated lymphoid tissue through the M cells and encounters naive T cells. If the Th1 pathway is operating correctly, the body will generate an immune response consisting of specific antibodies or specifically sensitised T lymphocytes. These T cells that become specialised for innocuous dietary antigens will in future act as a suppressive mechanism to prevent hypersensitivity developing via the Th2 pathway. This mechanism is known as oral tolerance.

However, if the antibody generated by the immune response is an IgE antibody, it will respond to further exposure to the antigen by generating an inflammatory reaction, which is the allergy. The mechanism of this type of allergy can be explained as follows: the IgE antibodies appear on the surface of cells, including circulating basophils. When the allergen/IgE interaction occurs, the cells presenting the IgE/allergen couple generate and release chemical mediators, including histamine. This phenomenon leads in pathologic effects, such as local or systemic vasodilatation.

The Th1 pathway is stimulated by the presence of bacteria. If a newly born or very young baby does not meet enough bacteria as may happen in developed countries with high standards of hygiene, the Th1 pathway may not function well. In such cases, the only response from the immune system will be a Th2 response and an allergy to the antigen in question will develop. This is only one cause of failure to develop oral tolerance; there may be others. Certainly there is thought to be a genetic predisposition.

Thus, the phenomenon of oral tolerance is the ability by which administration of antigens by the oral route can prevent subsequent systemic immune responses to the same antigen given in an immunogenic form. A similar mechanism is believed to prevent hypersensitivity responses to the normal bacterial flora of the intestine, which are also essential for life and are a source of harmless antigens. If the mechanism of oral tolerance does not develop sufficiently in an infant, or if there is a breakdown in the physiological state of tolerance to certain antigens, this may result in the development of hypersensitivity reactions.

Usually, milk hypersensitivity appears when a susceptible infant first encounters cows' milk. From a dietary point of view there are two ways to treat an established allergy - either foods containing the allergen must be avoided altogether, or the foods must be treated to decrease their allergenicity, for example by hydrolysis. Both infant formula and cereals containing extensively hydrolysed proteins (peptides consisting of not more than five amino acids) are manufactured for this latter purpose.

However, there is a need for products that help to reduce the risk of developing the allergy and promote the development of tolerance to intact proteins, particularly in children thought to be at risk of the same (for example if they have at least one family member suffering from an allergy). For example, it has been proposed to feed partially hydrolysed proteins to induce oral tolerance in infants. An alternative approach for the induction and maintenance of oral tolerance is described in WO 03/099037, namely the use of foods containing probiotic bacteria. Examples of probiotic bacteria include various species of *Lactobacilli* and *Bifidobacteria.* It has been found that administration of probiotics can help to induce oral tolerance in infants.

An object of the present invention is to provide a further approach to the promotion of oral tolerance.

Infant formulae often contain whey proteins which are of particular interest from a nutritional point of view, containing all essential amino acids and being quickly and easily digested.

Mizumachi et al., in Bioscience Biotechnology and Biochemistry, vol. 66, 2002, 1287-1294 disclose that "previous studies of oral tolerance to BLG (beta-lactoglobulin) have been reported by oral administration of BLG, whey protein, whey hydrolysate formulas or milk powder".

When whey proteins are heated in the presence of reducing sugars, free amino groups of the proteins will react with the sugars resulting in glycation of the proteins. If such reactions are allowed to proceed unchecked, the result can be a substantial reduction in nutritional value and some browning may be also be observed. The complex series of reactions that can occur are known collectively as the Maillard reaction. Indeed, it is even thought that Maillard type reactions may occur, albeit very slowly, in food products containing the necessary chemical groups at room temperature. Although the Maillard reaction is generally thought of as undesirable and steps are therefore taken to control it during processing of the relevant foodstuffs, more recently it has been realised that, carefully controlled, glycation of whey proteins might offer the opportunity to manipulate the properties of the proteins in various ways.

The first irreversible product resulting from the non-enzymatic interaction of a glycosyl group and the α- or ε- amino groups of proteins is known as an Amadori compound. All Amadori compounds generate furosine when subjected to acid hydrolysis and accordingly a method of monitoring the progress of glycation of milk proteins based on measurement of furosine production was devised. With this tool and the subsequent development of mass spectrometry techniques, it became theoretically possible to monitor the progress of the Maillard reaction. Glycation can be carried out in solution or in the solid state.

WO 00/18249 describes a process for the solid state glycation of powdered whey protein-containing materials comprising adjusting the water activity of the powder to 0.3 to 0.8 and allowing glycation to proceed at a temperature of 30 to 75 °C for between 1 hour and 80 days. It is claimed that the resulting powder has enhanced functional properties, such as enhanced heat stability, emulsifying activity, antioxidant activity and enterotoxin binding capacity. The powder may be used as an additive to enhance the functionality and nutritional content of foodstuffs.

EP-A-1514482 relates to nutritional and pharmaceutical compositions for treating and preventing cyclooxygenase-2 mediated diseases and to a process for increasing the cyclooxygenase-2 inhibiting activity of a product. As milk fractions found to exhibit an increased COX-2 inhibiting activity, whey fractions of mammalian milk are mentioned. E.g. acid whey fractions, sweet whey fractions, soluble whey fractions and combinations thereof may be used. It is further described that heating of milk proteins in the presence of lactose yields lactosylated proteins. After protection, the compounds will provide an increased resistance against degradation, in particular a proteolytic degradation in the body which in turn generally results in an increased bioavailability.

US-A-5750176 relates to the milk of a transgenic non-human mammal. The transgenic milk may also be used in the production of specialized enteral nutritional products. Also disclosed is a product produced in the milk of transgenic non-human mammals wherein the product results from the action of a catalytic entity selected from the group consisting of heterologous enzymes and heterologous antibodies, and wherein said transgenic non-human mammal contains in its genome at least one heterologous gene encoding for said catalytic entity. Examples of the aforementioned product are oliogosaccharides and glycoconjugates.

### Summary of the invention

The invention relates to a food product comprising glycated proteins for use in the promotion of oral tolerance to the unglycated proteins in a mammal, in accordance with claim 1. Such a product is simple to manufacture and administer and has nutritional value.

The glycated proteins are glycated whey proteins which comprise β-lactoglobulin. An example of a food product comprising glycated whey proteins is a baby or infant formula.

Alternatively the food product may be a dry, moist or liquid pet food comprising glycated whey proteins.

The glycated whey proteins comprise glycated β-lactoglobulin, and the glycation is lactosylation, i.e. the reducing sugar is lactose. Since these components are present in many food ingredients, for example whey protein isolate, whey protein concentrate or skimmed milk powder, it is simple to adapt the processes for the production of these products to include a lactosylation step. These whey protein-containing products are all widely used as food ingredients valued for the functional properties of their proteins as well as their good nutritional properties.

A method of producing a food product as described above comprises subjecting the proteins contained in the product to a glycation process. Glycation may be carried out either in solution or in the solid state.

In a further aspect, the invention relates to an infant formula containing glycated whey proteins, according to claim 2.

### Figures

Figure 1 shows anti-β-lactoglobulin IgE hyporesponsiveness in nine groups of mice fed with different types of whey protein or β-lactoglobulin with different extents of lactosylation;
Figure 2 shows anti-β-lactoglobulin IgG 1 hyporesponsiveness in the same nine groups of mice as Fig. 1;
Figure 3 shows anti-β-lactoglobulin IgE hyporesponsiveness in five groups of mice fed with different types of whey protein or β-lactoglobulin with different extents of lactosylation;
Figure 4 shows anti-β-lactoglobulin IgG1 hyporesponsiveness in the same five groups of mice as Fig.3.

### Detailed description of the invention

According to the invention, whey proteins which comprise β-lactoglobulin, which are antigens to which allergies may be developed, can be lactosylated and administered to a mammal in order to promote oral tolerance to said antigens.

By promotion of oral tolerance is meant both the induction and maintenance of oral tolerance together or separately.

The most common food allergy or hypersensitivity is cows' milk allergy or cows' milk hypersensitivity, since in most cases this is the first food product encountered by infants. Accordingly discussion herein is mostly centred on this allergy. However, the scope of the present patent application is not limited to this allergy, but is intended to relate to the promotion of oral tolerance with respect to other dietary whey proteins. Glycated whey proteins are preferably prepared by subjecting the proteins to a glycation process, preferably a solid state glycation process. According to Morgan (J Agric Food Chem (1999) 47(11):4543-8), solid state processes result in less conformational change of the protein molecule. Further, solid state processes are easier to monitor and control enabling undesirable effects, such as protein aggregation and advanced Maillard reactions, to be avoided.

The glycation process may comprise a mild heat treatment (50 - 80 °C) of the protein/sugar powders with an adjusted A_{w} (0.2-0.7) for up to about 72 hours. The overall process is very simple, well adapted to the existing industrial equipment and has a low-energy cost. The overall process may comprise adjustment and equilibration of the water activity (A_{w}) of the powders, heat treatment of the humidified powder; and optionally cooling down and/or drying of the powder.

Glycated proteins are comprised in a food product according to the claims. In the context of the present invention, the term "food product" is intended to encompass any consumable matter. Hence, it may be a product intended for consumption by humans, in particular infant formula, baby formula, infant and baby follow-up formula, and the like.

In accordance with Article 1.2 of the European Commission Directive 91/321/EEC of 14 May 1991 on infant formulae and follow-on formulae a child under the age of 12 months is considered an "infant". An "infant formula" is a foodstuff that is intended for the complete nutrition of infants during the first six months of life.

However, glycated whey proteins can also be included in food products not specifically intended for infant or baby nutrition but nevertheless consumed by this group, for example milk, yoghurt, curd, cheese, fermented milks, milk-based fermented products, ice-creams, fermented cereal based products, or milk-based products, among others. The term "food product" also encompasses products to be consumed by other mammals, for example pets, such as dogs, cats, rabbits, guinea pigs, mice, rats, or other domesticated mammals, such as livestock, for example, cattle, horses, pigs, sheep, goats, buffaloes, camels, and the like. Pets are in this respect preferably companion animals. Accordingly, the aforementioned glycated proteins can also be incorporated in pet food products in order to promote oral tolerance. Suitable pet food products include chunk products, croquettes, chew products, moist products, such as canned foods, and liquid formulas, such as milk for kittens or puppies.

Pet food is food suitable for consumption by pets and preferably foodstuff that is suitable for the complete nutrition of pets.

By definition, as the degree of glycation advances, the nutritional quality of the glycated protein is progressively reduced. For whey proteins, this may be estimated by measuring the % of blocked lysine. Limited glycation of these proteins does not result in noticeable detrimental effects on their nutritional value but the extent of glycation and the proportion of glycated protein used in a particular product must both be borne in mind when formulating the product. For example, an infant formula should preferably not contain more than about 15% blocked lysine. If glycated proteins are used in the formula, this target may be met either by glycating all the proteins to this maximum desired extent or by using a mixture of more highly glycated proteins and untreated proteins to respect overall the target for blocked lysine.

A method of producing such a food product is provided, comprises subjecting protein to a glycation process, preferably a solid state glycation process.

An infant formula containing glycated whey proteins can be of a standard composition with the proteins therein glycated or substituted with glycated proteins of choice. As discussed above, an infant formula containing glycated whey proteins should preferably not contain more than about 15% blocked lysine and this target may be met either by glycating all the proteins in the formula to this maximum desired extent or by using a mixture of more highly glycated proteins and untreated proteins to respect overall the target for blocked lysine.

The glycated proteins should preferably be consumed as a substitute to the unglycated protein until tolerance to the protein has been established.

An infant formula containing glycated whey proteins should be fed to a baby until weaning is complete and the child has reached the age of eating normal foods e.g. between 3 to 5 years.

### Examples

The following examples are illustrative of the products and methods of making the same falling within the scope of the present invention. They are not to be considered in any way limitative of the invention. Changes and modifications can be made with respect to the invention. That is, the skilled person will recognise many possible variations in these examples covering a wide range of compositions, ingredients, processing methods, and mixtures, and can adjust the naturally occurring levels of the compounds of the invention for a variety of applications.

### Example 1: Glycation of whey proteins in liquid conditions

β-Lactoglobulin (BLG) was obtained from Davisco (USA). The purity of (90 %) was assessed by RP-HPLC. Lactose was obtained from Fluka.

4% BLG (approximately 2.2 mM) and 7% lactose monohydrate (approximately 200 mM) were dissolved in 20 mM phosphate buffer, pH 6.8. After filtration on 0.22 mm acetate cellulose filters (Millipore), mixtures of protein and lactose were put in well-capped flasks and heated in a water bath at 60 °C for 0, 24 or 72 hours to reach low levels of lactosylation which do not induce noticeable detrimental effects on the nutritional value of the proteins. Theoretically these conditions allowed the binding of 2-3 lactose molecules per β-lactoglobulin monomer on a total of 14 possible lysine

residues (Morgan et al, 1999, J. Agri. Food Chem. 47, 83-91). All experiments were performed in anaerobic and sterile conditions by reducing the headspace to 1-2 ml in a 300 ml flask. After heating, the different fractions were not dialyzed but were immediately freeze-dried to prevent gelation.

The control native β-lactoglobulin (control BLG) was hydrated for 6 hours at room temperature, cooled in an ice-water bath, then lactose was added to reach the same 7% concentration as the glycated BLG.

As a further control the same purified BLG solution was incubated without lactose, cooled, then lactose powder was added and dissolved to reach the 7% concentration. Immediately after the solution was freeze-dried.

In addition two whey protein isolates (WPI) were tested:
- Davisco WPI BIPRO which is virtually depleted in lactosyl β-lactoglobulin (controlled by mass spectrometry), and
- Lacprodan 80 from Arla Foods which contained a high level of lactosyl β-lactoglobulin forms (characterized by mass spectrometry).

Blocked lysine was determined by the furosine assay. An aliquot of the protein (ca. 20 mg of protein) was hydrolyzed with 60 ml of 6 M hydrochloric acid for 24 hours at 110 °C in a reflux apparatus. After quantitative transfer to 100 ml volume with water, a 10 ml aliquot was evaporated to dryness and further resuspended in 3 ml of 0.02 M HCl. Analysis was then performed by injecting 10 µl in a Hitachi L-8500 amino acid analyser equipped with sodium citrate buffers and column for protein hydrolysate analysis. Lysine and furosine were quantified in the same chromatogram by comparison with external standards after post-column derivatization with ninhydrin reagent. The percentage of blocked lysine was calculated from the lysine and furosine content in the acid hydrolysates assuming that during hydrolysis about 40 % of ε-deoxyfructosyllysine (blocked lysine) is retransformed into lysine, part is transformed into pyridosine and about 32 % into furosine (Finot et al. (1981) Prog. Food Nutr. Sci. 5, 345-355). All results are mean values resulting from two independent measurements.

The results are shown in Table 1 below.

**Table 1**

| For group⁺ | Protein | % Blocked lysine |
|---|---|---|
| B | Control BLG, lyophilized with lactose | 42* |
| C | BLG + lactose T 0 | 3 |
| D | Control BLG, 24h incubation then lyophilized with lactose | 3 |
| E | BLG + lactose, 24h incubation then lyophilisation | 8 |
| F | Control BLG, 72h incubation then lyophilized with lactose | 5 |
| G | BLG + lactose, 72h incubation then lyophilisation | 14 |
| H | Commercial product WPI 95 Bipro | 1 |
| I | Commercial product Lacprodan 80 | 14 |
| + Mouse group to which the protein was fed in Example 2. | | |
| * This very high-value is due to a problem during lyophilisation. | | |

### Example 2: Induction of Oral Tolerance in Mice

To test the induction of oral tolerance, the standard mouse protocol described by Pecquet et al. (1999, Immunology 96: 278-285) was used. The mice were fed with different types of whey proteins or BLG, lactosylated or not, as described in Example 1. Five days later, mice were challenged by subcutaneous injections of BLG (100 mg β-lactoglobulin/mouse) diluted in a solution of Al(OH)₃. Three weeks later, mice were sacrificed and blood samples were collected in order to evaluate the oral tolerance induction by measuring titres of BLG-specific IgE and IgG1 in the different groups.

The mice were treated according to the following groups:
Group A: Water
Group B: Control BLG lyophilized with lactose (no incubation)
Group C: BLG + lactose T 0
Group D: Control BLG, 24h incubation then lyophilized with lactose
Group E: BLG + lactose, 24h incubation then lyophilisation
Group F: Control BLG, 72h incubation then lyophilized with lactose
Group G: BLG + lactose, 72h incubation then lyophilisation
Group H: WPI (Whey protein isolate) low lactosylation (Bipro)
Group 1: WPI high lactosylation (Lacprodan)

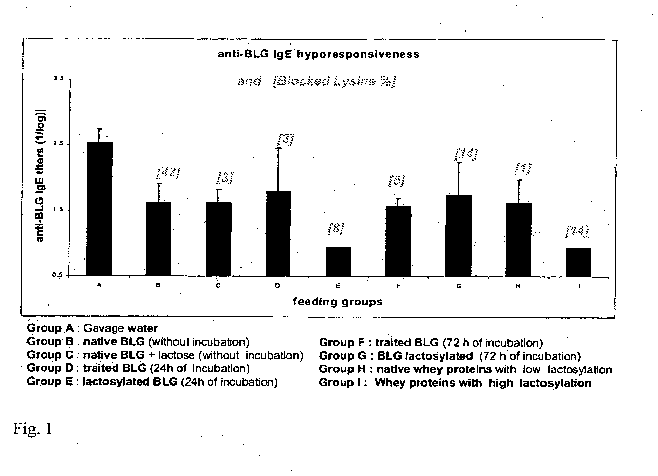

The results presented in Fig. 1 show that feeding the mice with lactosylated BLG (group E) and whey proteins with high lactosylation (group 1) further improves the specific anti-BLG IgE hyporesponsiveness when compared to the group that have been fed with native BLG (group B) and with whey proteins with low lactosylation (group H), both groups B and H being positive controls.

It is of note that group B (42% blocked lysine) did not produce the best response, despite the proteins fed having the highest rate of lactosylation. An explanation of this could be that the lactosylation reaction was not controlled. Further, it could be due to the fact that excessive lactosylation masks critical epitopes in the protein. In the same way, the results of group G are no better than the results from group I despite the same level of blocked lysine in the proteins fed to both of these groups. This may be because the lactosylation of the BLG given to group G was carried out in the liquid state and consequently was not well controlled. Liquid state lactosylation being difficult to control may lead to the occurrence of some undesirable phenomenon, including protein aggregation and advanced Maillard reaction. On the other hand, the lactosylation of the whey proteins given to the group I occurred in the dry state with better control (only early Maillard reaction).

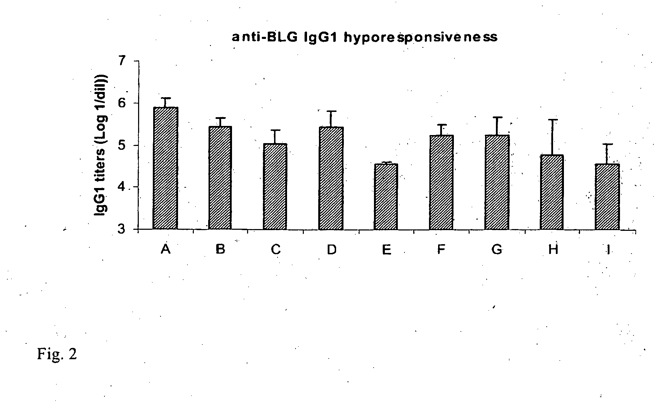

A similar profile was observed with anti-BLG specific IgG1, another isotype produced under the regulation of Th1 cells, showing the anergy of these cells due to oral tolerance induction. As shown Fig. 2, groups E and I present the lowest titre of anti-BLG IgG1, indicating that the lactosylation of the proteins in these two groups improves the hyporesponsiveness in comparison to the groups which were fed the same proteins with less lactosylation, i.e. groups B and H respectively.

### Example 3: Glycation in dry state

Prolacta 75 whey protein concentrate (WPC) was purchased from Lactalis Industrie (Retiers, France). According to the manufacturer, it contains 76.4 % protein, 15.3 % lactose, 3.2 % ash, 0.42 % calcium and 5 % water.

The WPC was lactosylated via solid state glycation. Briefly, the water activity (Aw) was adjusted to about 0.44 by placing the powder in a closed chamber with saturated K₂CO₃. Water uptake and Aw were monitored during this phase. When the required Aw was reached, the powder was sealed in opaque bags. The powder was then kept at least for 48 hours at 15° C to allow water to equilibrate within the matrix. Protein glycation was induced by incubating the powder at 60 °C for 3 h and 8 h in sealed stainless steel tubes (16 mm int. diam., ∼ 30 g powder) in a homemade tubule heating apparatus. Water activity was checked at the end of the glycation period, and no changes were observed. The powder was then stored hermetically at 15 °C.

Mass spectrometry (MALDI) was used to detect lactosylated forms of proteins and to quantify the average number of lactose linked per protein monomer in the Prolacta 75 (unglycated and glycated).

Glycation extend was evaluated by free amino group determination as described above.

Free amino groups were determined using the ortho-phthaldialdehyde method (OPA). The OPA determination was carried out by mixing 200 ml of the protein solution (1.5 g/l in 50 mmol/l sodium phosphate buffer pH 7.8), 2 ml of reducing solution (480 mg of N-acetyl-L-cystein dispersed in 200 ml of 0.1 mol/l sodium borate buffer pH 9.3) and 50 ml of a 20 % (w/w) SDS solution. After mixing and incubation for 10 minutes at 50 °C, 50 ml of the OPA reagent (prepared by dispersing 170 mg of OPA in 5 ml of methanol) were added. The mixture was further incubated 30 minutes at 50 °C and then cooled down at room temperature for 30 minutes before reading absorbance at 340 nm using an Uvikon 810 spectrophotometer (Flowspek, Basel, Switzerland). The calibration curve was obtained using an L-leucine standard in a concentration range of 10 to 200 mmol/l in final mixture. To obtain the signal of the reaction, two blanks were subtracted. Blank 1 (protein signal) was obtained with methanol instead of OPA reagent. Blank 2 was obtained by replacing the protein solution by the sodium phosphate buffer. Products and reagents used were respectively: boric acid, N-acetyl-L-cystein, OPA from Fluka (Chemie GmbH, Buchs, Switzerland), sodium dihydrogen phosphate monohydrate, methanol, sodium hydroxide from Merck (Darmstadt, Germany) and L-leucine from Sigma (St-Louis, MO, USA).

Size exclusion chromatography was used to check the presence of aggregated protein in the Prolacta 75 (unglycated and glycated). Protein samples were diluted with an adequate amount of elution buffer to give a protein concentration equivalent to 1 g/l based on the starting protein concentration. The column was a TSK 2000SW (Tosohaas), eluted with 0.1 M phosphate pH 6.8 + NaCl 0.15 M at a 0.5 ml/min. The optical density was recorded at 214 or 280 nm. The column was calibrated with MW standards containing native α-lactalbumin and β-lactoglobulin.

The results of the chemical characterisations are shown in Table 2 below.

**Table 2**

| | NH₂/Ntot OPA | Blocked lysine (Furosine) | Av. number of lactose bound per protein monomer (MALDI-MS) | | |
|---|---|---|---|---|---|
| Sample | | | BLG A | BLG B | α-LA |
| Prolacta 75 unglycated | 8.6 % | 5 % | 0.8 | 0.8 | 0.3 |
| Prolacta 75 3h glycation | 6.2 % | 19.7% | 3.4 | 3.6 | 2.6 |
| Prolacta 75 8h glycation | 5.5 % | 31.0 % | 5.4 | 5.7 | 3.4 |

A good correlation was found between the various methods used to characterize protein lactosylation. The conditions used for protein lactosylation (glycation in the dry state) did not lead to the formation of protein aggregates (as shown by size exclusion chromatography).

### Example 4: Induction of Oral Tolerance in Mice

In order to measure the effect of lactosylation, the induction of oral tolerance was performed in the mouse model as described above. The mice were fed with the different preparation of proteins unglycated and glycated Prolacta prepared in Example 3. Mice were then challenged at a systemic level with the β-lactoglobulin (BLG) and three weeks later the induction of oral tolerance to BLG was measured by evaluating the level of BLG specific IgE and IgG1 in the serum of the mice.

The mice were treated according to the following groups:
- Group A:: Lacprodan
- Group B:: Unglycated Prolacta 75
- Group C:: Glycated Prolacta 75 (blocked Lys: 19%)
- Group D:: Glycated Prolacta 75 (blocked Lys: 31 %)
- Group E:: Control (Water)

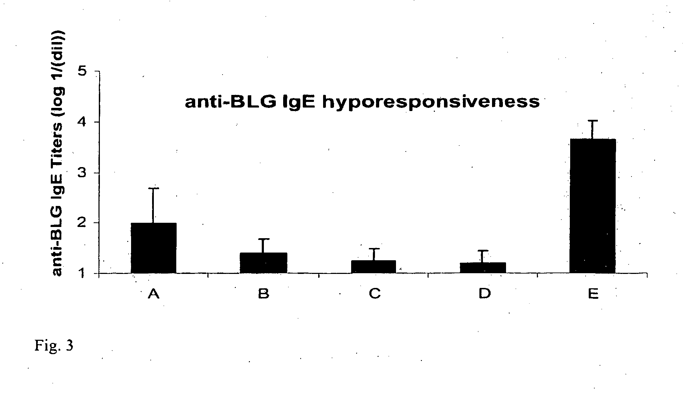

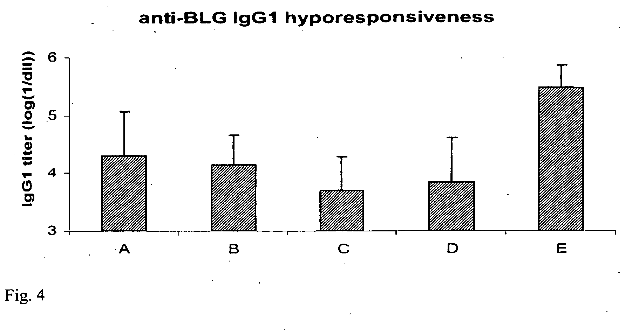

The results are shown in Fig. 3. In the same way as the results presented in Example 2, one can observe that the mice tolerised with the lactosylated Prolacta (groups C and D) show a better BLG specific IgE hyporesponsiveness than the groups fed un-lactosylated Prolacta or fed Lacprodan. The anti-BLG IgG 1 titres shown in Fig. 4 indicate the same type of tolerogenic response, groups C and D titres being the lowest.

In conclusion, this data shows that the lactosylation of whey proteins improves the specific tolerogenic hyporesponsiveness (IgE and IgG1), when compared to non-lactosylated proteins.

### Example 5: Infant Formula containing Glycated Whey Proteins

The composition of the infant formula is given below:-

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mag) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |

The protein source is Prolacta 75 whey protein concentrate glycated by the process described in Example 3 above to a blocked lysine content of 11 %. The infant formula is prepared by blending together the protein source, the carbohydrate source, and the fat source. Emulsifiers may be included in the blend if desired. Water which has been subjected to reverse osmosis is then be mixed in to form a liquid mixture.

The liquid mixture is thermally treated to reduce bacterial loads by rapidly heating it to a temperature in the range of about 80°C to about 110°C for about 5 seconds to about 5 minutes. The liquid mixture is then flash cooled to between 60°C and 85°C. The liquid mixture is then homogenised in two stages, first at about 7 MPa to about 40 MPa and then at about 2 MPa to about 14 MPa in the second stage. The homogenised mixture is further cooled and heat sensitive vitamins and minerals are added. The pH and solids content of the homogenised mixture is standardised at this point.

The homogenised mixture is then transferred to a spray drier and converted to powder having a moisture content of less than about 5% by weight.

## Claims

1. A food product comprising glycated proteins for use in the promotion of oral tolerance to the unglycated proteins in a mammal, wherein the glycated proteins are whey proteins, wherein the glycated whey proteins are lactosylated and wherein the whey proteins comprise β-lactoglobulin.

2. A food product as claimed in claim 1, wherein the food product is a baby or infant formula or a pet food.

## Patentansprüche

1. Nahrungsmittelprodukt, das glykierte Proteine umfasst, zur Verwendung zur Förderung einer oralen Toleranz gegenüber den nicht glykierten Proteinen bei einem Säuger, wobei die glykierten Proteine Molkeproteine sind, wobei die glykierten Molkenproteine laktosyliert sind und wobei die Molkeproteine β-Lactoglobulin umfassen.

2. Nahrungsmittelprodukt nach Anspruch 1, wobei das Nahrungsmittelprodukt eine Baby- oder Kleinkind-Nahrungsformulierung oder eine Haustiernahrung ist.

## Revendications

1. Produit alimentaire comprenant des protéines glyquées pour une utilisation dans la promotion de la tolérance orale aux protéines non glycosylées chez un mammifère, dans lequel les protéines glyquées sont des protéines lactosériques, dans lequel les protéines lactosériques glyquées sont lactosylées et dans lequel les protéines lactosériques comprennent de la β-lactoglobuline.

2. Produit alimentaire selon la revendication 1, dans lequel le produit alimentaire est une préparation pour bébés ou pour nourrissons ou un aliment pour animaux de compagnie.
